# EUROPÄISCHE PATENTSCHRIFT

(11) **EP 1 205 475 B1**
(45) Veröffentlichungstag und Bekanntmachung des Hinweises auf die Patenterteilung: **27.10.2004**
(21) Anmeldenummer: 01126541.0
(22) Anmeldetag: 14.11.2001
(51) Int. Cl.: C07D 311/30, C07D 405/12, A61K 31/352, A61K 7/40, A61P 17/16, A23L 1/30

(54) **Flavonoidverbindungen zur Verwendung gegen oxidativen Stress und UV-Strahlung**
Flavonoid compounds for use against oxidative stress and UV radiation
Composés flavonoides utilisés contre le stress oxydatif et les rayons UV

(30) Priorität: 14.11.2000 DE 10056400
(43) Veröffentlichungstag der Anmeldung: 15.05.2002
(73) Patentinhaber: MERCK PATENT GmbH, 64293 Darmstadt (DE)
(72) Erfinder: Pfluecker, Frank, Dr., 64297 Darmstadt (DE); Buenger, Joachim, Dr., 64823 Gross-Umstadt (DE); Driller, Hans-Juergen, Dr., 64823 Gross-Umstadt (DE); Buchholz, Herwig, Dr., 60599 Frankfurt (DE); Rosskopf, Ralf, 64839 Muenster (DE)
(74) Vertreter: Isenbruck, Günter, Dr.

(56) Entgegenhaltungen:
- DE-A- 19 508 608
- FR-A- 2 687 572
- US-A- 5 665 367

## Beschreibung

Die Erfindung betrifft Verbindungen der Formel I 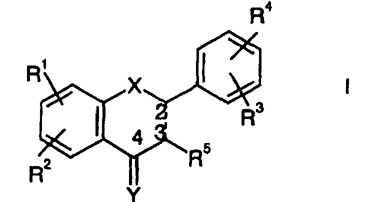 wobei X, Y, R¹, R², R³, R⁴ und R⁵ die in Anspruch 1 angegebene Bedeutung aufweisen.

Eine mehr oder minder stark ausgeprägte Sonnenbräune der Haut gilt in der modernen Gesellschaft als attraktiv und als Ausdruck von Dynamik und Sportlichkeit. Neben dieser erwünschten Wirkung der Sonne auf die Haut treten eine Reihe von unerwünschten Nebenwirkungen auf, wie Sonnenbrand oder vorzeitige Hautalterung und Faltenbildung. Inzwischen sind eine Anzahl von leistungsfähigen UV-Filtern entwickelt worden, die in Form von Cremes, Lotionen oder Gelen auf die Haut aufgetragen auch bei stärkerer Sonneneinwirkung die Entwicklung von Sonnenbrand wirksam verhindern können. Der in der pharmazeutischen oder kosmetischen Zubereitung enthaltene UV-Filter bildet auf der Oberfläche der Haut einen Film bzw. eine Schicht aus und dringt nicht mit weiteren in der Zubereitung enthaltenen pflegenden Substanzen in tiefere Hautschichten vor. Bekannte UV-Filter bzw. Sonnenschutzmittel wirken also nur in der Weise, daß sie bestimmte Bereiche des Sonnenlichts absorbieren und somit diese Strahlung nicht in tiefere Schichten der Haut vordringen kann. Bekanntlich wird der gefährlichste Teil der Sonnenstrahlung von den ultravioletten Strahlen mit einer Wellenlänge von weniger als 400 nm gebildet. Die untere Grenze der ultravioletten Strahlen, welche die Erdoberfläche erreichen, ist durch die Absorption in der Ozonschicht auf ca. 280 nm beschränkt. Die heute in der Kosmetik üblichen Sonnenschutzfilter absorbieren in einem Wellenlängenbereich von 280 bis 400 nm. Dieser Bereich umfaßt UV-B-Strahlen mit einer Wellenlänge zwischen 280 und 320 nm, die bei der Bildung eines Sonnenerythems eine entscheidende Rolle spielen, wie auch UV-A-Strahlen, mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische und irritative Reaktionen auslösen können.

Hautschädigungen werden nicht nur durch Sonnenlicht verursacht, sondern auch durch andere äußere Einflüsse, wie Kälte oder Wärme. Ferner unterliegt die Haut einer natürlichen Alterung, wodurch Falten entstehen und die Spannkraft der Haut nachläßt.

Aufgabe pflegender Kosmetik ist es, nach Möglichkeit den Eindruck einer jugendlichen Haut zu erhalten. Prinzipiell stehen verschiedene Wege offen, um diesen Weg zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen. Die Idee ist also, vorbeugend einzugreifen und dadurch den Alterungsprozess hinauszuzögern. Ein Beispiel sind hierfür die bereits erwähnten UV-Filter, welche durch Absorption bestimmter Wellenlängenbereiche eine Schädigung der Haut vermeiden oder zumindest vermindern. Während bei UV-Filtern das schädigende Ereignis, die UV-Strahlung, von der Haut abgeschirmt wird, versucht man bei einem weiteren Weg, die natürlichen Abwehr- bzw. Reparaturmechanismen der Haut gegen das schädigende Ereignis zu unterstützen. Schließlich verfolgt man als weiteren Ansatzpunkt die mit zunehmendem Alter sich abschwächenden Abwehrfunktionen der Haut gegen schädigende Einflüsse auszugleichen, indem Substanzen von außen zugeführt werden, die diese nachlassende Abwehr- bzw. Reparaturfunktion ersetzen können. Beispielsweise besitzt die Haut die Fähigkeit, Radikale, die durch äußere oder innere Stressfaktoren erzeugt werden, abzufangen. Diese Fähigkeit schwächt sich mit zunehmendem Alter ab, wodurch sich der Alterungsprozess mit zunehmendem Alter beschleunigt.

Substanzen, welche Abwehr- und Reparaturfunktionen der Haut unterstützen oder ersetzen sollen, müssen an ihren Wirkungsort transportiert werden können. Dazu stehen prinzipiell zwei Wege offen. Entweder wird die Substanz auf die Haut aufgetragen und dringt durch die äußeren Schichten in tiefere Schichten der Haut vor oder der Wirkstoff wird, z.B. nach oraler Aufnahme, über die Blutbahn zum Wirkort transportiert.

Eine weitere Schwierigkeit bei der Herstellung von Kosmetika besteht darin, daß Wirkstoffe, die in kosmetische Formulierungen eingearbeitet werden sollen, oftmals nicht stabil sind und in der Formulierung geschädigt werden können. Die Schädigungen können beispielsweise durch eine Reaktion mit Luftsauerstoff oder durch die Absorption von UV-Strahlen verursacht werden. Die so geschädigten Moleküle können durch ihre Struktur-änderung z.B. ihre Farbe ändern und/oder ihre Wirksamkeit verlieren.

In der DE 195 08 608 wird ein nicht beständiges kosmetisches Mittel zum Schutz vor UV-Strahlen der Wellenlänge zwischen 280 und 400 nm beschrieben, welches mindestens ein Tetraalkylquercetin in einem kosmetisch annehmbaren Medium auf Ölbasis enthält, sowie neue Tetraalkylquercetine.

In der FR 2 687 572 wird die Verwendung von Flavonoiden, die keine Doppelbindung an der Position 2, 3 bzw. nicht gleichzeitig eine Doppelbindung an der Position 2, 3 und eine Hydroxylgruppe an der Position 3 aufweisen, für die Herstellung von kosmetischen Zusammensetzungen sowie als Wirkstoffe zum Schutz der Haut, der Haare oder von kosmetischen Zusammensetzung gegen Singulettsauerstoff beschrieben.

In der EP 0 716 847 A1 wird eine kosmetische oder dermatologische Wirkstoffkombination aus Zimtsäurederivaten und Flavonglykosiden beschrieben. Die Wirkstoffkombination wirkt dabei als Antioxidanz. Sie eignet sich zur Bekämpfung oder Prophylaxe der Hautalterung. Die Wirkstoffkombination kann als weiterer Bestandteil auch UV-Filtersubstanzen umfassen.

In der US 5,665,367 wird eine Zusammensetzung für die Hautpflege beschrieben, welche Retinol oder einen Retinylester sowie ein Flavonoid, das ausgewählt ist aus der Gruppe, die aus Naringenin und Quercetin besteht, die einen geeigneten kosmetischen Träger enthält. Die Zusammensetzung kann als weitere Komponente auch UV-Filter enthalten.

In der FR 2 708 851 wird eine kosmetische Zusammensetzung beschrieben, die Nanopigmente sowie als Antioxidationsmittel Flavonoide enthält. Als weiterer Inhaltsstoff können auch UV-A- oder UV-B-Filter enthalten sein.

Aufgabe der Erfindung ist es, Verbindungen zur Verfügung zu stellen die sowohl als Antioxidationsmittel wirken als auch einen Schutz vor UV-Strahlung bewirken können.

Diese Aufgabe wird gelöst durch Verbindungen der Formel I 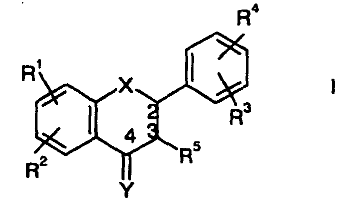 wobei X steht für O, S oder NH;
Y steht für O, S oder NH;
zwischen den Kohlenstoffen C-2 und C-3 eine Einfach- oder eine Doppelbindung vorgesehen sein kann,
wobei R¹, R², R³, R⁴ und R⁵ gleich oder verschieden sein können und unabhängig voneinander stehen für -H-OH oder -OA, und A steht für eine Gruppe, die im UV-A- und/oder UV-B-Bereich UV-Strahlung absorbiert, welche ausgewählt ist aus der Gruppe, die gebildet ist aus 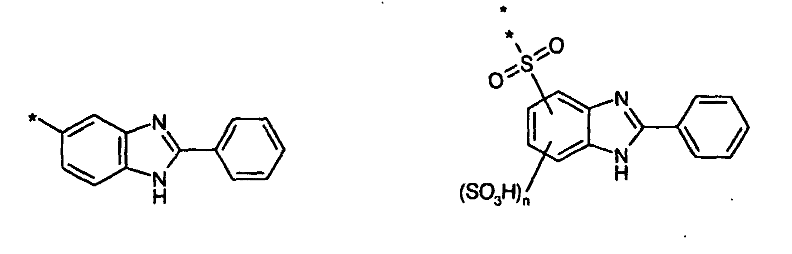 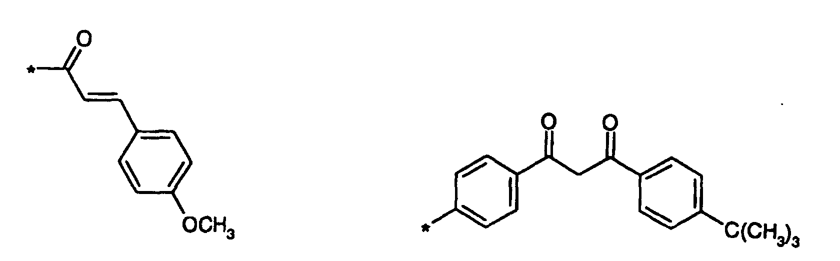 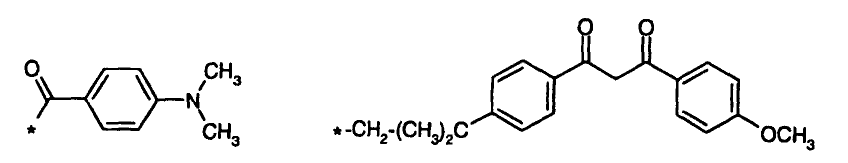 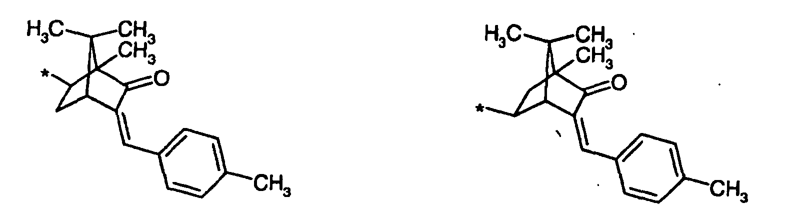 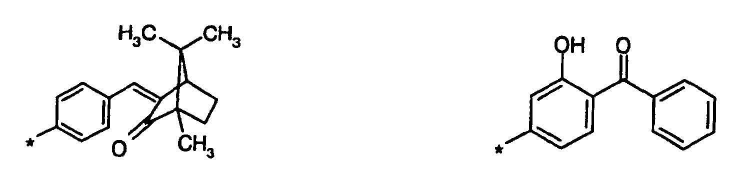 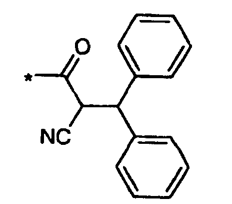 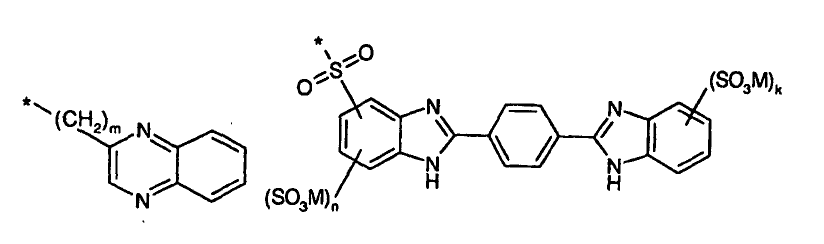 mit
n = 0, 1, 2 oder 3
m = 0 oder 1
k = 0, 1, 2, 3 oder 4
M = H, Na oder K
und zumindest eine der Gruppen R¹, R², R³, R⁴ oder R⁵ durch -OA gebildet ist.

Bevorzugt weisen die Verbindungen eine Struktur der Formel II auf 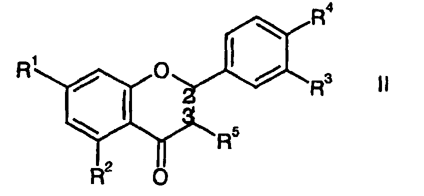 wobei R¹, R², R³, R⁴ und R⁵ die oben bzw. im weiteren angegebene Bedeutung haben.

Die den Grundkörper der Verbindungen der Formel I bildenden Flavonoide zeigen eine Wirkung als Antioxidationsmittel bzw. als Radikalfänger. Durch die Kombination mit Gruppen, die eine geeignete Absorption im UV-Bereich zeigen, steht eine neue Verbindungsklasse zur Verfügung, die sehr interessante Eigenschaften aufweist.

Neben ihrer Wirkung als Antioxidanz zeigen die als Grundkörper verwendeten Flavonoide bereits eine gewisse Schutzwirkung gegen UVA-Strahlung. Durch die Kombination mit UV-absorbierenden Gruppen können daher UVA/B-Breitbandfilter zur Verfügung gestellt werden, die gleichzeitig antioxidative Wirkung zeigen. Die Gruppen werden dabei so ausgewählt, daß sie insbesondere in den für die Haut besonders schädlichen Wellenlängenbereichen der UVA- und der UVB-Strahlung ein Absorptionsmaximum zeigen. Der UVB-Anteil des Sonnenlichts umfaßt den Bereich von 280 bis 320 nm und die UVA-Strahlung den Bereich von > 320 nm, welches sich direkt an den Bereich des sichtbaren Lichts anschließt. Derartige UV-Breitbandfilter können beispielsweise in einer Vielzahl von Kosmetika zum Einsatz gelangen.

Durch ihre Wirkung als Antioxidanz wirken Verbindungen der Formel I auch stabilisierend auf Formulierungen, die für Kosmetika verwendet werden. Durch Beigabe von Verbindungen der Formel I zu den entsprechenden Produkten bleiben diese daher länger stabil und verändern ihr Aussehen nicht. Insbesondere bleibt auch bei länger dauernder Anwendung bzw. längerer Lagerung die Wirksamkeit der Inhaltsstoffe, z.B. Vitamine, erhalten. Dies ist besonders vorteilhaft bei Sonnenschutzmitteln, da diese Kosmetika besonders hohen Belastungen durch UV-Strahlen ausgesetzt sind.

Allgemein wirken die Substanzen der Formel I als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalanionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Die Verbindungen der Formel I wirken auch als Enzymhemmer. So hemmen sie Histidindecarboxylase, Proteinkinasen, Elastase, Aldosereduktase sowie Hyaluronidase, was ermöglichen würde, die Unversehrtheit der Grundsubstanz vaskulärer Hüllen aufrecht zu erhalten. Ferner hemmen sie nicht spezifisch Katechol-O-methyltransferase, wodurch die Menge der verfügbaren Katecholamine und dadurch die Gefäßfestigkeit erhöht werden kann. Als weitere Wirkung hemmen sie AMP-Phosphodiesterase, wodurch die Substanzen ein Potential zur Hemmung der Thrombozytenaggregation aufweisen können.

Diese Eigenschaften ermöglichen beispielsweise eine Verwendung von Verbindungen der Formel I für eine Vielzahl von therapeutischen Anwendungen. Sie sind beispielsweise geeignet zum Schutz der DNA. Weitere Verwendungen ergeben sich aus ihrer Wirkung als antivirales Mittel insbesondere als Mittel gegen Herpes. Ferner können sie als bakteriostatisches oder bakteriozides Mittel oder auch als antiinflammatorisches Mittel verwendet werden.

Verbindungen der Formel I werden meistens nicht in Substanz angewendet, sondern in geeignete Formulierungen, wie Cremes, Salben oder Lotionen eingearbeitet. Eine Anwendung der Verbindungen kann durch Auftragen auf die Haut erfolgen, aber auch ein systemischer Transport ist denkbar. Dazu müssen die Verbindungen der Formel I in ihren Eigenschaften an die jeweiligen Bedingungen angepaßt werden. Eine Modifikation der Öl- oder Wasserlöslichkeit ist durch die Einführung entsprechender Gruppen in das Molekül möglich. Als geeignet haben sich Verbindungen gezeigt, wobei weiter R¹, R², R³, R⁴ und R⁵ unabhängig voneinander stehen für eine
- geradkettige oder verzweigte Oxalkyl- oder Carboxyalkylgruppe mit 1 bis 12 Kohlenstoffatomen,
- geradkettige oder verzweigte Oxalkenyl- oder Carboxyalkenylgruppe mit 2 bis 12 Kohlenstoffatomen,
- geradkettige oder verzweigte Hydroxyoxalkylgruppe mit 1 bis 12 Kohlenstoffatomen, wobei die Hydroxylgruppe an ein primäres oder sekundäres Kohlenstoffatom gebunden sein kann und weiter die Alkylkette auch durch Sauerstoff unterbrochen sein kann,
- Sulfatgruppe,
- Phosphatgruppe,
- sowie für einen Mono- oder Oligoglycozylrest,
wobei diese Gruppen durch Alkyl-, Hydroxy-, Alkoxy-, Amino-, Mono- und Dialkylamino-, Sulfonsäure-, Carboxyl- und/oder Halogengruppen substituiert sein können.

Zur Verbesserung der Öllöslichkeit werden die Hydroxylgruppen des Flavonoidgrundkörpers mit geeigneten Alkoholen verethert. Eine besonders geeignete Alkylgruppe ist die 2-Ethylhexylgruppe.

Die Öllöslichkeit läßt sich auch verbessern, indem beispielsweise die Hydroxygruppen der Flavonoidgrundkörper mit 2-Ethylhexancarbonsäure verestert werden.

Weitere geeignete Reste werden durch Veresterung mit Monocarbonsäuren, wie Buttersäure, Valeriansäure, Hexansäure, Sorbinsäure, Ascorbinsäure oder Laurinsäure erhalten. Durch diese Reste wird der Transport der Verbindung durch biologische Membranen gefördert. In der Zelle kann der Grundkörper dann freigesetzt werden, indem durch Esterasen die Carbonsäuren abgespalten werden.

Die Löslichkeit in Wasser kann verbessert werden, indem Sulfat- oder Phosphatgruppen in das Molekül eingeführt werden. Geeignet sind sowohl Verbindungen mit einer, zwei oder drei Sulfatgruppen im Molekül, wie auch Gemische verschiedener Sulfate bzw. Phosphate.

Bevorzugt werden auch solche Strukturen ausgewählt, bei denen zumindest einer der Reste R¹, R², R³, R⁴ und R⁵ durch ein Mono- oder Oligosaccharid gebildet wird. Bevorzugt sind dabei Hexosylreste, insbesondere Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch vorteilhaft sein, Pentosylreste zu verwenden. Die Glucosylreste können α- oder β-glycosydisch mit dem Grundkörper verbunden sein. Ein bevorzugtes Disaccharid ist beispielsweise das 6-O-(6-Deoxy-α-L-mannopyranosyl)-β-D-glucopyranosid.

Die Herstellung der Verbindungen der Formel I erfolgen nach Verfahren, die dem Fachmann an sich bekannt sind und in Standardwerken wie Houben-Weyl, "Methoden der organischen Chemie", Georg-Thieme-Verlag Stuttgart, beschrieben sind.

Die Einführung der Reste kann z.B. ausgehend von den bekannten Flavonoidgrundkörpern erfolgen. So können Alkylketten durch Reaktion der Grundkörper mit den entsprechenden Alkylhalogeniden unter Wirkung einer starken Base in einem geeigneten Lösungsmittel, beispielsweise Dimethylformamid, eingeführt werden. Erfolgt eine Anbindung der Reste R¹ bis R⁵ als Ester, kann dies beispielsweise durch Umsetzung mit einem entsprechenden Säurechlorid erfolgen. Stellvertretend sind in Schema 1 einige Vorstufen der Reste angegeben, die für eine Einführung der Reste in dem jeweiligen Grundkörper geeignet sind. -X steht dabei für eine geeignete Abgangsgruppe, beispielsweise ein Halogenid. Diese Aufstellung ist nur beispielhaft und zeigt nur einen kleinen Ausschnitt aus der Menge der möglichen Vorstufen. Dem Fachmann ist es ohne weiteres möglich, andere geeignete Abgangsgruppen, beispielsweise eine Tosylatgruppe oder eine Triflatgruppe anstelle der Halogenide vorzusehen. Beispielsweise ist eine Einführung der über eine Esterfunktion gebundenen Reste auch durch entsprechende Umesterungsreaktionen möglich. 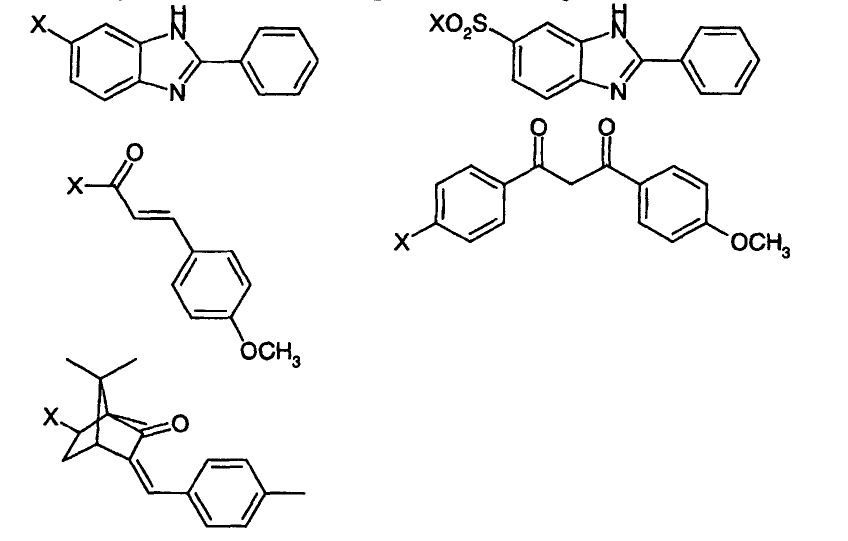 Schema 1: Beispiele für geeignete Vorstufen zur Einführung von Resten R¹, R², R³, R⁴ und R⁵

Die Verbindungen der Formel I können entweder als reine Verbindungen verwendet werden. Es ist jedoch auch die Verwendung von Gemischen möglich, wobei darunter sowohl Isomerengemische als auch Gemische von Verbindungen der Formel I verstanden werden, die unterschiedliche Substitutionsmuster aufweisen, also beispielsweise eine unterschiedliche Anzahl von Substituenten-OA tragen.

Die Umsetzung der Flavonoidgrundkörper mit den entsprechenden reaktiven Vorstufen der Reste R¹ bis R⁵ erfolgt nach dem Fachmann bekannten Verfahren und in den üblichen Lösungsmitteln. Geeignete Lösungsmittel sind beispielsweise chlorierte Kohlenwasserstoffe, Dimethylsulfoxid, Dimethylformamid usw.

Die Verbindungen der Formel I können eine vorteilhafte Wirkung auf die Haut entfalten. Gegenstand der Erfindung ist deshalb auch eine kosmetische oder pharmazeutische Formulierung enthaltend zumindest eine Verbindung der Formel I.

Die positive Wirkung der erfindungsgemäßen kosmetischen oder pharmazeutischen Zubereitung beruht vermutlich auf der Wirkung der Verbindungen der Formel I als Antioxidationsmittel bzw. Radikalfänger in Kombination mit einer Wirkung als UV-Filter. Um ihre positive Wirkung auf die Haut entwickeln zu können, sollten die Verbindungen der Formel I in tiefere Hautschichten vordringen können. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar. Die Zubereitung wird dann beispielsweise so gestaltet, daß sie für eine orale Gabe geeignet ist.

Die eine oder die mehreren Verbindungen der Formel I können in der üblichen Weise in kosmetische Formulierungen eingearbeitet werden. Für eine innerliche Anwendung sind Darreichungsformen wie Kapseln, Dragees, Pulver, Tabletten-Lösungen oder Lösungen geeignet.

Als Darreichungsform der erfindungsgemäßen kosmetischen oder pharmazeutischen Formulierungen für eine äußerliche Anwendung seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Formulierung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isoethionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isoethionate, lmidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Alle Verbindungen oder Komponenten, die in den kosmetischen Formulierungen verwendet werden können, sind entweder bekannt und käuflich erwerbbar oder können nach bekannten Verfahren synthetisiert werden.

Die erfindungsgemäße kosmetische Zubereitung eignet sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrig-alkoholischer Gele bzw. Lösungen, als feste Stifte vorliegen oder als Aerosol konfektioniert sein.

Die Formulierung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den Verbindungen der Formel I beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße kosmetische Zubereitung kann auch als alkoholisches Gel vorliegen, welches einen oder mehrere Niedrigalkohole oder - polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.
Ist eine Zubereitung als Aerosol konfektioniert, verwendet man in der Regel die üblichen Treibmittel, wie Alkane, Fluoralkane und Chlorfluoralkane.

Die kosmetische Formulierung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Formulierung vor oder nach dem Shamponieren, vor oder nach dem Färben oder Entfärben bzw. vor oder nach der Dauerwelle aufgetragen wird. Es kann auch eine Formulierung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die kosmetische oder pharmazeutische Formulierung kann außer der oder den Verbindungen der Formel I verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäßen kosmetischen Zubereitungen können mit Hilfe von Techniken hergestellt werden, die dem Fachmann wohl bekannt sind.

Zum Schutz der Haut und/oder natürlicher oder sensibilisierter Haare vor Sonnenstrahlen wird auf die Haut oder die Haare eine kosmetische Zubereitung, enthaltend eine oder mehrere Verbindungen der Formel I aufgetragen. Als sensibilisierte Haare werden dabei Haare verstanden, welche einer chemischen Behandlung, wie einer Dauerwellenbehandlung, einem Färbe- oder Entfärbeprozeß unterzogen worden sind.

Die schützende Wirkung gegen UV-Strahlung kann verbessert werden, wenn die Formulierung einen oder mehrere UV-Filter enthält.

Prinzipiell kommen alle UV-Filter für eine Kombination in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie
- 3-(4'-Methylbenzyliden)-dl-kampfer (z.B. Eusolex® 6300),
- 3-Benzylidenkampfer (z.B. Mexoryl® SD),
- Polymere von N-{(2 und 4)-[(2-oxoborn-3-yliden)methyl]benzyl}acrylamid (z.B. Mexoryl® SW),
- N,N,N-Trimethyl-4-(2-oxoborn-3-ylidenmethyl)anilinium methylsulfat (z.B. Mexoryl® SK) oder
- α-(2-Oxoborn-3-yliden)toluol-4-sulfonsäure (z.B. Mexoryl® SL),

Benzoyl- oder Dibenzoylmethane wie
- -1-(4-tert-Butylphenyl)-3-(4-methoxyphenyl)propan-1,3-dion (z.B.
- Eusolex® 9020) oder
- -4-lsopropyldibenzoylmethan (z.B. Eusolex® 8020),

Benzophenone wie
- 2-Hydroxy-4-methoxybenzophenon (z.B. Eusolex® 4360) oder
- 2-Hydroxy-4-methoxybenzophenon-5-sulfonsäure und ihr Natriumsalz (z.B. Uvinul® MS-40),

Methoxyzimtsäureester wie
- Methoxyzimtsäureoctylester (z.B. Eusolex® 2292),
- 4-Methoxyzimtsäureisopentylester, z.B. als Gemisch der Isomere (z.B. Neo Heliopan® E 1000),

Salicylatderivate wie
- 2-Ethylhexylsalicylat (z.B. Eusolex® OS),
- 4-Isopropylbenzylsalicylat (z.B. Megasol®) oder
- 3,3,5-Trimethylcyclohexylsalicylat (z.B. Eusolex® HMS),

4-Aminobenzoesäure und Derivate wie
- 4-Aminobenzoesäure,
- 4-(Dimethylamino)benzoesäure-2-ethylhexylester (z.B. Eusolex® 6007),
- ethoxylierter 4-Aminobenzoesäureethylester (z.B. Uvinul® P25),
und weitere Substanzen wie
- 2-Cyano-3,3-diphenylacrylsäure-2-ethylhexylester (z.B. Eusolex® OCR),
- 2-Phenylbenzimidazol-5-sulfonsäure sowie ihre Kalium-, Natrium- und Triethanolaminsalze (z.B. Eusolex® 232),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin ( z.B. Uvinul® T 150)
- 4,4-Diarylbutadiene.

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1 - 8 %, in kosmetische Formulierungen eingearbeitet.

Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- α-(Trimethylsilyl)-ω-[(trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1) ,
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 187 393-00-6).

Diese organischen UV-Filter werden in der Regel in einer Menge von 0,5 bis 20 Gewichtsprozent, vorzugsweise 1 - 15 %, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusolex®T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Formulierungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxyphenyl)-propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze.

Die organischen bzw. anorganischen UV-Filter können beliebig mit den Verbindungen der Formel I kombiniert werden. Bevorzugt wird bezogen auf das Gewicht das Verhältnis der UV-Filter zu den Verbindungen der Formel I zwischen 1:10 und 10:1, insbesondere zwischen 1:5 und 5:1 gewählt.

Durch Kombination von einer oder mehrerer Verbindungen der Formel I mit weiteren UV-Filtern kann die Schutzwirkung gegen schädliche Einwirkungen der UV-Strahlung optimiert werden. Die Kombination zeigt dann sowohl Schutzwirkung als Antioxidationsmittel als auch vor Verbrennungen durch UV-Strahlung.

Die schützende Wirkung gegen oxidativen Stress bzw. gegen die Einwirkung von Radikalen kann weiter verbessert werden, wenn die Formulierung ein oder mehrere weitere Antioxidantien enthält.

Es gibt viele aus der Fachliteratur bekannte und bewährte Substanzen, die verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajaretsäure, Trihydroxybutyrophenon, Quercetin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Formulierungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbinsäure und Zitronensäure (z.B. Oxynex® L LIQUID), DL-α-Tocopherol, Zitronensäure und Lecithin (z.B. Oxynex® LM) oder Butylhydroxytoluol (BHT), L-(+)-Ascorbylpalmitat und Zitronensäure (z.B. Oxynex® 2004).

Die Antioxidationsmittel können beliebig mit den Verbindungen der Formel I kombiniert werden. Bevorzugt wird bezogen auf das Gewicht das Verhältnis des Antioxidationsmittels zu den Verbindungen der Formel I zwischen 1:10 und 10:1, insbesondere zwischen 1:5 und 5:1 gewählt.

Die erfindungsgemäßen Formulierungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Formulierungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die erfindungsgemäße Formulierung kann weiter als Inhaltsstoff auch Ectoin (2-Methyl-1,4,5,6-tetrahydropyrimidin-4-carbonsäure) enthalten und bewirkt dann einen Schutz der Langerhanszellen. Durch die Zugabe von 1-(2-Hydroxyphenyl)-alkan-1-on-oxim erhält die erfindungsgemäße Formulierung eine antiinflammatorische Wirkung.

Die kosmetische oder pharmazeutische Formulierung enthaltend mindestens eine Verbindung der Formel I eignet sich besonders zum Schutz von Körperzellen gegen oxidativen Stress, insbesondere zur Verringerung der Hautalterung.

Ebenso positiv sind die Eigenschaften von Verbindungen mit der Formel I zu werten für eine Verwendung in Nahrungsmitteln oder als Nahrungsergänzungsmittel oder als "functional food". Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren Verbindungen der Formel I angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen). Nahrungsmittel, die nach der vorliegenden Erfindung mit einer oder mehreren Verbindungen der Formel I angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensoße, Tomatenpüree usw. Weitere Beispiele für Nahrungsmittel, die nach der vorliegenden Erfindung mit einer oder mehreren Verbindungen der Formel I angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren Verbindungen der Formel I angereichert zu werden, beispielsweise Multivitaminpräparate, Mineralstoffmischungen oder gezuckerter Saft. Als weitere Beispiele für Nahrungsmittel, die nach der vorliegenden Erfindung mit einer oder mehreren Verbindungen der Formel I angereichert werden können, seien Nahrungsmittelzubereitungen, beispielsweise zubereitete Cerealien, Gebäck, Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die nach der vorliegenden Erfindung mit einer oder mehreren Verbindungen der Formel I angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die Nahrungsmittel, die nach der vorliegenden Erfindung mit einer oder mehreren Verbindungen der Formel I angereichert werden können, werden vorzugsweise oral angewendet, z.B. in Form von Speisen, Pillen, Tabletten, Kapseln, Pulver, Sirup, Lösungen oder Suspensionen.

Die mit einer oder mehreren Verbindungen der Formel I angereicherten erfindungsgemäßen Nahrungsmittel können mit Hilfe von Techniken hergestellt werden, die dem Fachmann wohl bekannt sind.

Durch ihre vorteilhafte Wirkung insbesondere als Antioxidationsmittel bzw. als Radikalfänger eignen sich Verbindungen der Formel I auch als Arzneimittel. Sie wirken dabei unterstützend oder substituierend zu natürlichen Mechanismen, welche Radikale im Körper abfangen. Die Verbindungen der Formel I können in ihrer Wirkung teilweise mit Radikalfängern wie Vitamin C verglichen werden. Verbindungen der Formel I können beispielsweise zur vorbeugenden Behandlungen von Entzündungen und Allergien der Haut sowie in bestimmten Fällen zur Verhütung bestimmter Krebsarten verwendet werden.

Insbesondere eignen sich Verbindungen der Formel I wegen ihrer antiallergischen, antiinflammatorischen und antiirritativen Wirkungen zur Herstellung eines Arzneimittels zur Behandlung von Entzündungen, Allergien und Irritationen, insbesondere der Haut.

Ferner können Arzneimittel hergestellt werden in einer Wirkung als Venentonikum, als Mittel zur Erhöhung der Festigkeit von Blutkapillaren, als Hemmstoff für Cuperose, als Hemmstoff chemischer, physikalischer oder aktinischer Erytheme, als Mittel zur Behandlung empfindlicher Haut, als Dekongestionsmittel, als Entwässerungsmittel, als Mittel zum Schlankmachen, als Antifaltenmittel, als Stimulatoren der Synthese von Komponenten der extrazellulären Matrix, als stärkendes Mittel zur Verbesserung der Hautelastizität und als Antialterungsmittel.

Ein weiterer Gegenstand der Erfindung betrifft die Stabilisierung von UV-Filtern. Eine bekannte und leistungsfähige Klasse von Lichtschutzfiltersubstanzen wird von den Dibenzoylmethanderivaten gebildet. Nachteilig ist jedoch, daß diese Substanzen sehr leicht durch UV-Licht zersetzt werden und damit ihre schützenden Eigenschaften verlorengehen. Als Beispiel für einen auf dem Markt erhältlichen Lichtschutzfilter aus dieser Verbindungsklasse sei das 4-(tert.-Butyl)-4'-methoxydibenzoylmethan angeführt, welches die unten gezeigte Struktur aufweist.

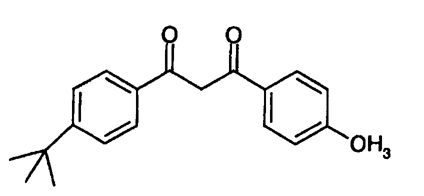

Überraschend hat sich nun gezeigt, daß Verbindungen mit der Formel I eine sehr gute Stabilisierungswirkung für die Dibenzoylmethane, insbesondere 4-(tert.-butyl)-4-methoxybenzoylmethan, aufweisen. Eine besonders hohe Stabilisierungswirkung wurde für eine Verbindung der Formel I gefunden, wobei X = -CH₂- und R¹ = R² = R³ = R⁴ = H ist. Indem Gemische dieser Verbindungen in Kosmetika eingearbeitet werden, ist es nun möglich, Lichtschutzmittel unter Verwendung von Dibenzoylmethanen herzustellen, die auch bei längerer Sonneneinwirkung, beispielsweise während eines mehrstündigen Sonnenbades, keine oder nur eine geringe Verringerung der Schutzwirkung gegen UV-Strahlen aufweisen.

Die Erfindung wird anhand von Beispielen näher erläutert.

### Beispiel 1:

### Einführung einer Schutzgruppe an Quercetin :

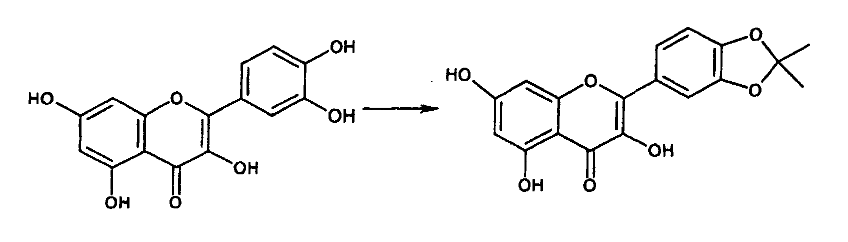

Eine Mischung aus Quercetin (604 mg, 2 mmol) und Diphosphorpentoxid (284 mg) in Dichlormethan (10 ml) wird auf 75°C erhitzt. Zu der Suspension gibt man tropfenweise Aceton (0,3 ml) und rührt für eine weitere Stunde bei 75°C. Nach der Entfernung eines öligen Rückstandes gibt man NaOH (25%, 1.5 ml) zur Reaktionsmischung. Die isolierte organische Phase wird anschliessend mit Wasser gewaschen und durch Entfernung des Lösungsmittels im Vakuum erhalten.

### Beispiel 2:

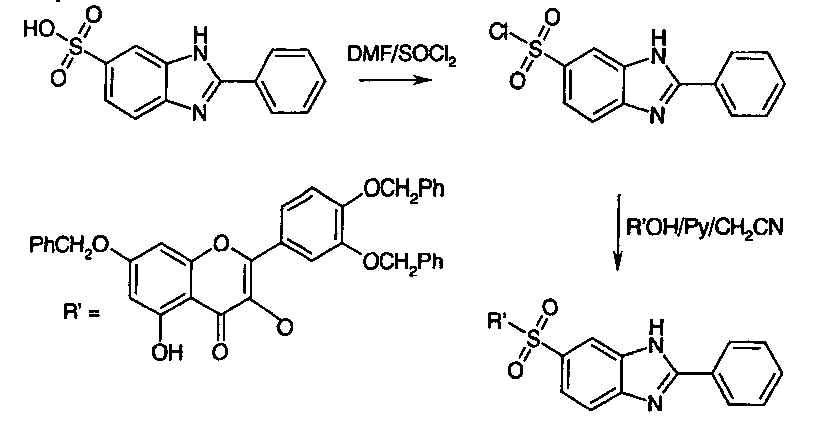

a) Chlorierung von 2-Phenylbenzimidazol-5-sulfonsäure :
   Dimethylformamidchlorid Synthese :
      Äquimolare Mengen Thionylchlorid und DMFA werden unter Kühlung zusammengegeben. Das Gemisch wird 30 min. bei Raumtemperatur stehengelassen und anschliessend unter Feuchtigkeitsausschluss im Wasserstrahlvakuum bei 30-40°C bis zur Gewichtskonstanz eingedampft. Man erhält einen schach gelb gefärbten Kristallbrei, der mit Ether gewaschen wird.
   Dimethylformamidchlorid mit 2-Phenylbenzymidazol-5-sulfonsäure :
      6.2 g Dimethylformamidchlorid und 5 g 2-Phenylbenzimidazol-5-sulfonsäure werden in 120 ml DMFA während 24 Std. bei 20°C zur Reaktion gebracht. Das Gemisch wird mit Eis versetzt und anschliessend mit Toluol extrahiert. Nach Entfernung des Lösungsmittels erhält man das gewünschte Produkt durch Umkristallisation aus CH₂Cl₂/Hexan.
b) Umsetzung von 2-Phenylbenzimidazol-5-sulfonylchlorid mit Acetongeschütztem Quercetin:
   2-Phenylbenzymidazol-5-sulfonylchlorid (2mol) und 3',4',7-Tribenzylquercetin (1 mol) werden unter Rückfluss in Toluol erhitzt. Die organische Phase wird anschliessend mit einer wässrigen Lösung aus Kaliumcarbonat gewaschen. Nach Separation der organischen Phase erhält man das gewünsche Produkt durch Entfernung des Lösungsmittels im Vakuum.

### Beispiel 3:

### Darstellung von 4-(Dimethylamino)benzosäure-2-ethylhexylester

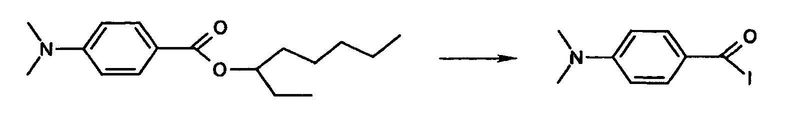

Zu einer Lösung von 4-(Dimethylamino)benzosäure-2-ethylhexylester (0.1 mol) in Chloroform (5 ml) gibt man Jod (0.1 mol) und Diiodosilan (0.1 mol). Die Reaktionsmischung wird anschliessend bei 50°C für 1.5 Stunde gerührt. Nach Entfernung des Lösungsmittels erhält man 4-(Dimethylamino)-2-acyliodid-benzol.

### Beispiel 4:

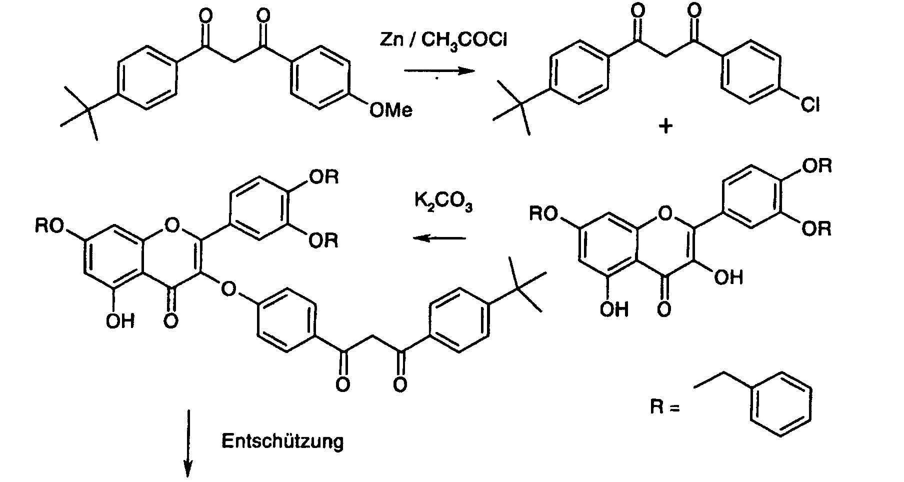

a) Chlorierung von 1-(4-Tert-butylphenyl)-3-(4-methoxyphenyl)propan-1,3-dion (Eusolex 9020)
   Eine Suspension von Zink (130 mg) in Ether (20 ml) wird mit 1-(4-Tert-butylphenyl)-3-(4-methoxyphenyl)propan-1,3-dion (2,48 g) und Acetylchlorid unter Schutzgastechnik versetzt und weitere 1.5 Stunden gerührt. Die Reaktionsmischung wird anschliessend mit Wasser gequencht. Nachdem festes Zink mittels Filtration entfernt wurde, wird der Reaktionsansatz mit Ether (5 x 5ml) gewaschen. Die vereinigten organischen Phasen werden mit Natriumcarbonat- sowie Natriumchloridlösung gewaschen und im Vakuum getrocknet.
b) Synthese des Ethers:
   Eine Mischung von 3',4',7-Tribenzylquercetin (3g), 1-(4-Tert-butylphenyl)-3-(4-chlorophenyl)propan-1,3-dion und K₂CO₃ (30 g) wird in getrockneten Aceton (300 ml) 21 Stunden unter Rückfluss erhitzt. Anschliessend wird die Lösung abgesaugt und mehrmal mit warmen Aceton gewaschen. Aus dem Filtrat erhält man durch Entfernung des Acetons im Vakuum das gewünschte Produkt.
c) Entschützung:
   3',4',7-Tribenzyl-2-(1-(4-tert-butylphenyl)-3-(4-oxophenyl)propan-1,3-dion)-Quercetin wird in 200 ml Methanol gelöst und mit Hilfe von 0.3 g 10%iger Pd-Kohle bei 1 bar hydriert. Nach Entfernung des Katalysators sowie des eingesetzten Lösungsmittels erhält man das gewünschte Produkt.

### Beispiel 5:

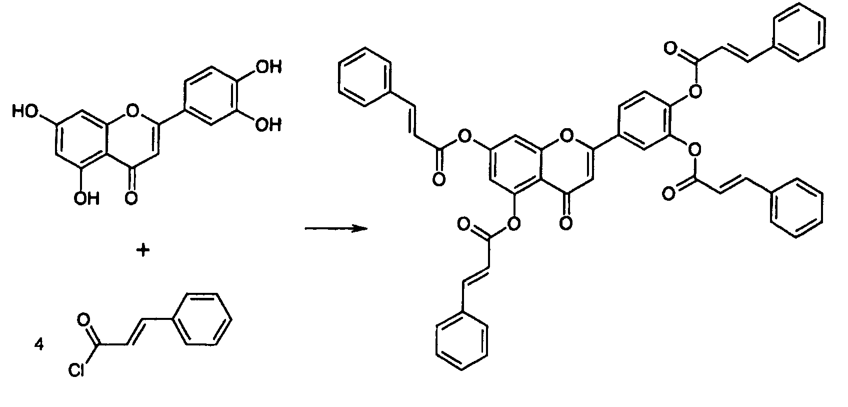

1 g Luteolin wurde in 50 ml Pyridin und 50 ml CH₂Cl₂ vorgelegt und bei Raumtemperatur 100 ml Zimtsäurechlorid, gelöst in 25 ml CH₂Cl₂ langsam zugetropft. Nach Beendigung der Zugabe wurde noch für weitere 3 Stunden bei Raumtemperatur gerührt. Anschließend wurden 100 ml H₂O und 50 ml CH₂Cl₂ zugegeben, die organische Phase abgetrennt und die wässrige Phase einmal mit CH₂Cl₂ extrahiert. Die vereinigten organischen Phasen wurden mit Wasser, 2N Salzsäure und wässriger Natriumcarbonatlösung gewaschen und anschließend getrocknet. Nach Abdestillieren des Lösungsmittels und Umkristallisieren aus Toluol wurden feine weiße Kristalle erhalten.

Ausbeute: 2,16 g (83 % d. Th.).

### Beispiel 6:

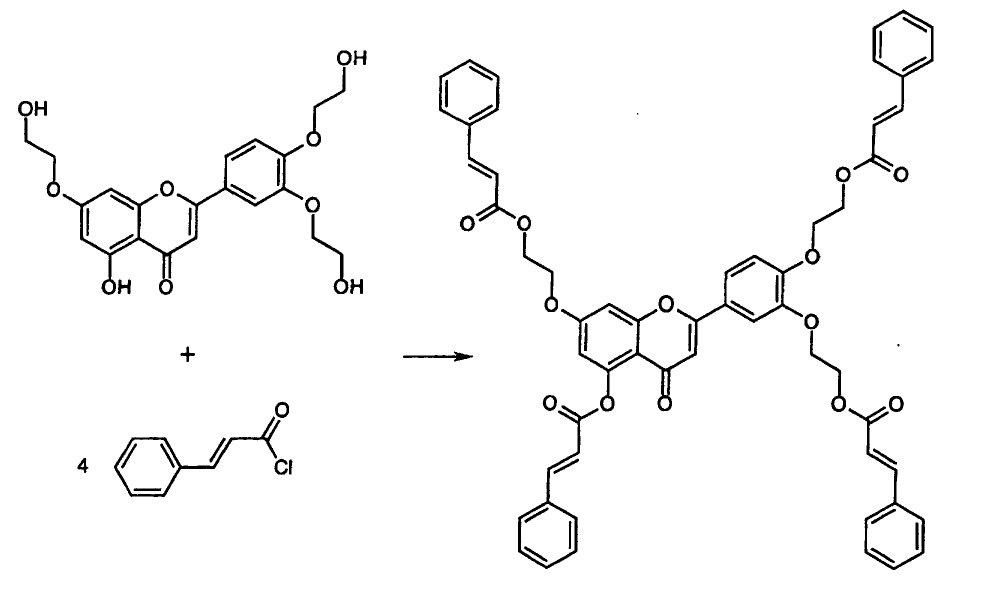

1 g Troxeluteolin und 50 ml Pyridin wurden in 70 ml CH₂Cl₂ vorgelegt. Es wurde langsam eine Lösung von 10 ml Zimtsäurechlorid in 50 ml CH₂Cl₂ bei Raumtemperatur zugetropft. Nach Beendigung der Zugabe wurde das Reaktionsgemisch noch für weitere 3 Stunden gerührt. Es wurden 50 ml Wasser zugegeben, die organische Phase abgetrennt und mit Wasser, 2N Salzsäure und erneut mit Wasser und dann wässriger Kalziumcarbonatlösung gewaschen. Die organische Phase wurde getrocknet und anschließend das Lösungsmittel unter vermindertem Druck abdestilliert. Umkristallisation aus Toluol ergab ein hellbeiges Pulver.

Ausbeute: 1,09 g (67,3 % d. Th.).

### Beispiel 7

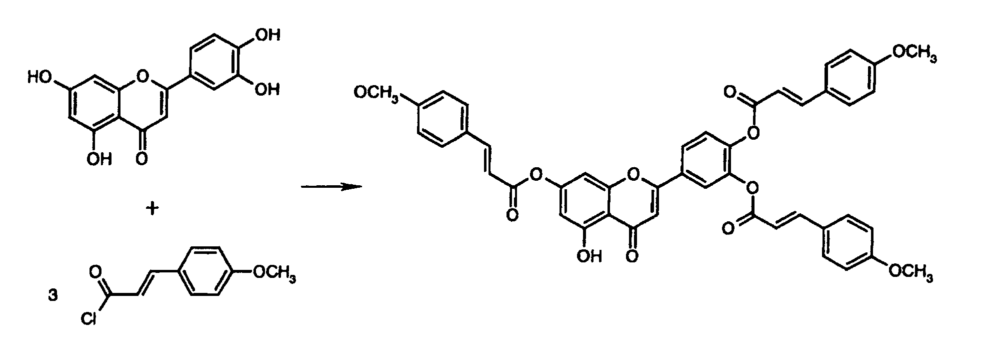

0,5 g Luteolin und 50 ml Pyridin wurden in 25 ml CH₂Cl₂ vorgelegt und 1.026 g trans-p-Methoxyzimtsäurechlorid in 25 ml CH₂Cl₂ langsam bei Raumtemperatur zugetropft. Nach beendeter Zugabe wurde die Reaktionsmischung noch für weitere 8 Stunden gerührt. Es wurden 100 ml Wasser und 200 ml CH₂Cl₂ zugegeben, die organische Phase abgetrennt und die wässrige Phase mit CH₂Cl₂ extrahiert. Die vereinigten organischen Phasen wurden mit Wasser, 2N Salzsäure und gesättigter Natriumchloridlösung gewaschen.

Das Lösungsmittel wurde unter vermindertem Druck abdestilliert. Umkristallisation aus Toluol ergab ein hellgelbes Pulver.

Ausbeute: 0,48 g (39 % d. Th.).

### Beispiel 8

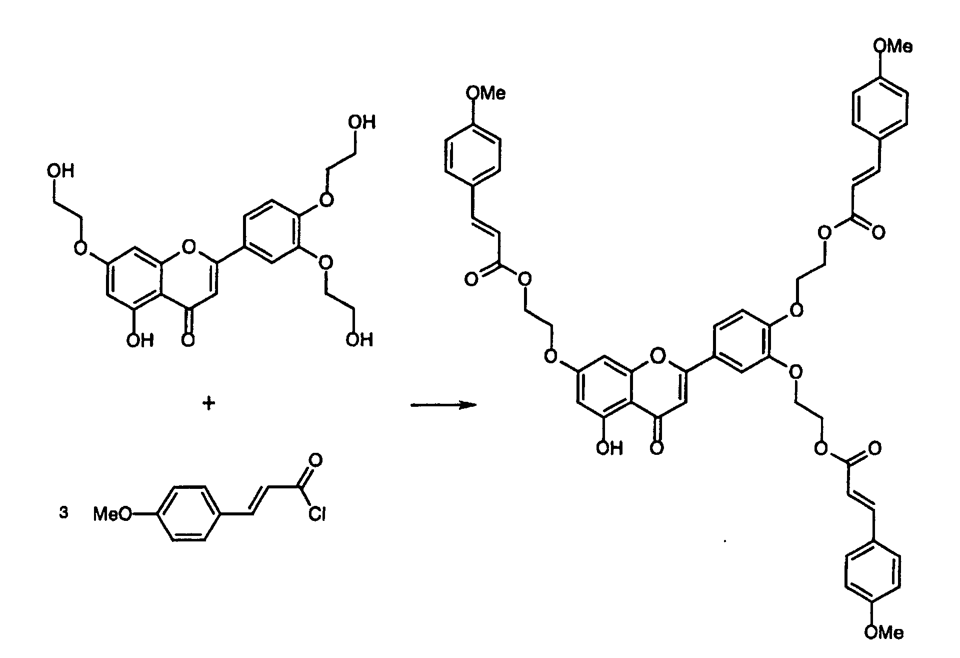

0,5 g Troxeluteolin und 25 ml Pyridin wurden in 20 ml CH₂Cl₂ vorgelegt und dann 0,9838 g trans-Methoxyzimtsäurechlorid in 20 ml CH₂Cl₂ bei Raumtemperatur langsam zugetropft. Nach beendeter Zugabe wurde die Mischung noch für weitere 8 Stunden gerührt. Es wurden 100 ml H₂O und 50 ml CH₂Cl₂ zugegeben, die organische Phase abgetrennt und die wässrige Phase mit CH₂Cl₂ extrahiert. Die vereinigten organischen Phasen wurden mit Wasser, 2N Salzsäure und erneut mit Wasser gewaschen. Nach Trocknen wurde das Lösungsmittel unter vermindertem Druck abdestilliert. Umkristallisation aus Toluol ergab ein schwach gelbes Pulver.

Ausbeute: 0,63 g (59 % d. Th.).

Im weiteren werden beispielhafte Zusammensetzungen kosmetischer Formulierungen angegeben.

Für die Herstellung der Zusammensetzungen werden die in den Beispielen 2 und 7 hergestellten Verbindungen verwendet. Dabei bedeutet "Verbindung 2" die in Beispiel 2 hergestellte Verbindung und "Verbindung 7" die in Beispiel 7 hergestellte Verbindung.

### Beispiel 1

| **Pflegelotion (W/O) zum Auftragen auf die Haut** | | **Gew.-%** |
|---|---|---|
| **A** | Verbindung 7 | 1,00 |
| | Polyglyceryl-2-Dipolyhydroxystearat | 5,00 |
| | Bienenwachs | 0,50 |
| | Zinkstearat | 0,50 |
| | Hexyllaurat | 9,00 |
| | Cetylisononanoat | 6,00 |
| | Shea Butter | 0,50 |
| | DL-α-Tocopherolacetat | 1,00 |
| | | |
| **B** | Glycerin | 5,00 |
| | Magnesiumsulfat-Heptahydrat | 1,00 |
| | Konservierungsmittel | q.s. |
| | Wasser, demineralisiert | ad 100,00 |

### Herstellung:

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:
0,05 % Propyl-4-hydroxybenzoat
0,15 % Methyl-4-hydroxybenzoat

### Beispiel 2

| **Pflegelotion (W/O) zum Auftragen auf die Haut** | | **Gew.-%** |
|---|---|---|
| **A** | Polyglyceryl-2-dipolyhydroxystearat | 5,00 |
| | Bienenwachs | 0,50 |
| | Zinkstearat | 0,50 |
| | Hexyllaurat | 9,00 |
| | Cerylisononanoat | 6,00 |
| | Shea Butter | 0,50 |
| | DL-α-Tocopherolacetat | 1,00 |
| | | |
| **B** | Verbindung 2 | 1,00 |
| | Glycerin | 5,00 |
| | Magnesiumsulfat-Heptahydrat | 1,00 |
| | Konservierungsmittel | q.s. |
| | Wasser, demineralisiert | ad 100,00 |

### Herstellung:

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:
0,05 % Propyl-4-hydroxybenzoat
0,15 % Methyl-4-hydroxybenzoat

### Beispiel 3

| **Hautcreme (O/W)** | | **Gew.%** |
|---|---|---|
| **A** | Paraffin | 2,00 |
| | Isohexadecan | 2,00 |
| | Isopropylpalmitat | 3,00 |
| | Sojaöl | 0,50 |
| | Dimeticon | 1,00 |
| | Ceryl Alkohol | 1,00 |
| | Sorbitolstearat | 1,50 |
| | Cerylalkohol (und) Cerylglucosid | 4,00 |
| | (-) -α- Bisabolol | 0,30 |
| | | |
| **B** | Wasser, demineralisiert | ad 100,00 |
| | Verbindung 2 | 1,00 |
| | Glycerin 87 % | 10,00 |
| | D-Panthenol | 0,50 |
| | (D+)-Biotin | 0,05 |
| | Konservierungsmittel | q.s. |
| | | |
| **C** | Xanthan Gummi | 0,30 |
| | | |
| **D** | Parfüm | 0,20 |

### Herstellung:

Phase A und Phase B werden getrennt auf 75°C erwärmt. Dann wird Phase C langsam zu Phase B gegeben und bis zur Homogenisierung gerührt. Die erhaltene Mischung B/C wird bei einer Temperatur von 75°C zu Phase A gegeben und die Mischung homogenisiert. Nach Abkühlen auf 35°C werden Parfümstoffe zugegeben.

Als Konservierungsmittel werden verwendet:
0,05 % Propyl-4-hydroxybenzoat
0,15 % Methyl-4-hydroxybenzoat
0,30 % Germall 115 (ISP, Frechen)

### Beispiel 4

| | | **Gew.%** |
|---|---|---|
| **A** | Verbindung 7 | 1,00 |
| | Paraffin | 2,00 |
| | Isohexadecan | 2,00 |
| | Isopropylpalmitat | 3,00 |
| | Sojaöl | 0,50 |
| | Dimeticon | 1,00 |
| | Cerylalkohol | 1,00 |
| | Sorbitolstearat | 1,50 |
| | Cerylalkohol (und) Cerylglucosid | 4,00 |
| | (-) -α- Bisabolol | 0,30 |
| | | |
| **B** | Wasser, demineralisiert | ad 100,00 |
| | Glycerin 87 % | 10,00 |
| | D-Panthenol | 0,50 |
| | (D+)-Biotin | 0,05 |
| | Konservierungsmittel | q.s. |
| | | |
| **C** | Xanthan Gummi | 0,30 |
| | | |
| **D** | Parfüm | 0,20 |

### Herstellung:

Phase A und Phase B werden getrennt auf 75°C erwärmt. Dann wird Phase C langsam zu Phase B gegeben und bis zur Homogenisierung gerührt.

Die erhaltene Mischung B/C wird bei einer Temperatur von 75°C zu Phase A gegeben und die Mischung homogenisiert. Nach Abkühlen auf 35°C werden Parfümstoffe zugegeben.

Als Konservierungsmittel werden verwendet:
0,05 % Propyl-4-hydroxybenzoat
0,15 % Methyl-4-hydroxybenzoat
0,30 % Germall 115 (ISP, Frechen)

### Beispiel 5

| **Sonnenschutzspray (O/W)** | | **Gew.-%** |
|---|---|---|
| **A** | Verbindung 7 | 1,00 |
| | Eusolex® 2292 (Art.-Nr. 105382) | 7,50 |
| | Eusolex® HMS (Art.-Nr. 111412) | 7,00 |
| | Steareth-2 | 0,40 |
| | Steareth-10 | 0,80 |
| | Pemulen® TR-2 | 0,18 |
| | Propylen-Glycol Isoceteth-3 Acetat | 5,00 |
| | Performa® V 825 | 0,80 |
| | Dimeticon | 1,00 |
| | Oxynex® K (Art.-Nr. 108324) | 0,10 |
| | | |
| **B** | Eusolex® 232 (Art. Nr. 105372) | 1,00 |
| | Triethanolamin | 0,90 |
| | Propandiol-1,2 | 2,00 |
| | Konservierungsmittel | 0,50 |
| | Wasser demineralisiert | a.d. 100,00 |
| Eusolex® 2292, Eusolex® HMS und Eusolex® 232 sind UV-Filter, die von der Merck KGaA, Darmstadt, vertrieben werden. | | |
| Pemulen®TR-2 ist ein Acrylat/C₁₀-C₃₀-Alkylacrylatpolymeres, das von der Fa. Goodrich, Neuss, vertrieben wird, | | |
| Performa® V825 ist ein synthetisches Wachs, das von der Fa. New Phase, NJ08554 vertrieben wird, | | |
| Oxynex® K ist eine Mischung aus PEG-8, Tocopherol, Ascorbylpalmitat, Ascorbinsäure und Zitronensäure und wird von der Merck KGaA, Darmstadt vertrieben. | | |

### Herstellung:

### Phase B:

Das Wasser wird mit dem Triethanolamin vermischt und anschließend Eusolex® 232 unter Rühren zugegeben. Sobald sich alles gelöst hat, werden die weiteren Bestandteile der Phase B zugegeben und anschließend die Mischung auf 80°C erwärmt.

### Phase A:

Die Bestandteile der Phase A mit Ausnahme von Pemulen® TR-2 werden zusammengegeben und auf 80°C erwärmt. Anschließend wird das Permulen® TR-2 unter Rühren zugegeben.

### Herstellung des Sonnenschutzmittels:

Zur Phase A wird unter Rühren langsam die Phase B gegeben. Nach dem Homogenisieren wird unter Rühren abgekühlt.

Als Konservierungsmittel wurden verwendet:
0,05 % Propyl-4-hydroxybenzoat
0,15 % Methyl-4-hydroxybenzoat

### Beispiel 6

| **Sonnenschutzspray (O/W)** | | **Gew.-%** |
|---|---|---|
| **A** | Eusolex® 2292 (Art.-Nr. 105382) | 7,50 |
| | Eusolex® HMS (Art.-Nr. 111412) | 7,00 |
| | Steareth-2 | 0,40 |
| | Steareth-10 | 0,80 |
| | Pemulen® TR-2 | 0,18 |
| | Propylen-Glycolisoceteth-3-acetat | 5,00 |
| | Performa® V 825 | 0,80 |
| | Dimeticon | 1,00 |
| | Oxynex® K (Art.-Nr. 108324) | 0,10 |
| | | |
| **B** | Verbindung 2 | 1,00 |
| | Eusolex® 232 (Art.-Nr. 105372) | 1,00 |
| | Triethanolamin | 0,90 |
| | Propandiol-1,2 | 2,00 |
| | Wasser, demineralisiert | ad 100,00 |

### Herstellung:

### Phase B:

Das Wasser wird mit dem Triethanolamin vermischt und anschließend Eusolex® 232 unter Rühren zugegeben. Sobald sich alles gelöst hat, werden die weiteren Bestandteile der Phase B zugegeben und anschließend die Mischung auf 80°C erwärmt.

### Phase A:

Die Bestandteile der Phase A mit Ausnahme von Pemulen® TR-2 werden zusammengegeben und auf 80°C erwärmt. Anschließend wird das Pemulen® TR-2 unter Rühren zugegeben.

### Herstellung des Sonnenschutzmittels:

Zur Phase A wird unter Rühren langsam die Phase B gegeben. Nach dem Homogenisieren wird unter Rühren abgekühlt.

Als Konservierungsmittel werden verwendet:
0,05 % Propyl-4-hydroxybenzoat
0,15 % Methyl-4-hydroxybenzoat

### Beispiel 7

| Sonnenschutzgel (wässrig) | | Gew.-% |
|---|---|---|
| **A** | Verbindung 7 | 1,00 |
| | Eusolex® 232 (Art.-Nr. 105372) | 4,00 |
| | Natriumhydroxidlösung | 6,00 |
| | Glycerin | 3,00 |
| | Propandiol-1,2 | 2,00 |
| | Konservierungsmittel | q.s. |
| | Wasser, demineralisiert | ad 100,00 |
| | | |
| **B** | Carbomer Ultrez-10 | 0,70 |
| | Wasser, demineralisiert | 60,00 |
| | | |
| **C** | Natriumhydroxidlösung (10 %) | 1,50 |
| | Wasser, demineralisiert | 4,00 |
| Carbomer Ultrez-10 wird von der Fa. Goodrich, Neuss, DE, angeboten. | | |

### Herstellung:

Carbomer Ultrez-10 wird im Wasser der Phase B vollständig dispergiert. Anschließend wird langsam die Phase C zugegeben und homogenisiert.

Für die Phase A wird zunächst das Wasser zur Natriumhydroxidlösung gegeben und dann das Eusolex® 232 zugegeben und unter Rühren vollständig gelöst. Nach Erhalt einer klaren Lösung werden die weiteren Bestandteile der Phase A zugegeben. Anschließend wird Phase A portionsweise zum Gemisch der Phasen B und C gegeben, wobei nach jeder Zugabe homogenisiert wird.

Als Konservierungsmittel wird verwendet:
0,20 % Methyl-4-hydroxybenzoat

### Beispiel 8

| **Sonnenschutzgel (O/W)** | | **Gew.-%** |
|---|---|---|
| **A** | Verbindung 7 | 1,00 |
| | Eusolex® 6300 (Art.-Nr. 5385) | 0,75 |
| | Luvitol® EHO | 10,00 |
| | | |
| | Dimeticon | 2,00 |
| | Sheabutter | 5,00 |
| | Antaron® V-220 | 2,00 |
| | Oxynex® K | 1,00 |
| | | |
| **B** | Eusolex® 232 (Art. Nr. 5372) | 0,75 |
| | Tris(hydroxymethyl)-aminomethan | 0,33 |
| | Konservierungsmittel | q.s. |
| | Wasser, demineralisiert | 20,00 |
| | | |
| **C** | Tris(hydroxymethyl)-aminomethan | 1,20 |
| | Wasser, demineralisiert | 10,00 |
| | | |
| **D** | Pemulen® TR-1 | 0,60 |
| | Wasser, demineralisiert | ad 100,00 |
| Pemulen®TR-1 ist ein Acrylat/Alkylacrylatpolymeres, das von der Firma Goodrich, Neuss, DE, vertrieben wird, | | |
| Eusolex® 6300 ist ein UV-Filter, der von der Firma Merck KGaA, Darmstadt, DE, vertrieben wird, | | |
| Luvitol® EHO wird von der Fa. BASF AG, Ludwigshafen, DE, vertrieben, | | |
| Antaron® V-220 wird von der Fa. GAF, Frechen, DE, vertrieben. | | |

### Herstellung:

Das Pemulen® TR-1 wird im Wasser der Phase D gelöst. Das Tris(hydroxymethyl)aminomethan wird im Wasser der Phase C gelöst und die Lösung zur Phase D gegeben. Das Tris(hydroxymethyl)aminomethan wird im Wasser der Phase B gelöst und dann unter Rühren das Eusolex® 232 zugegeben. Nach Erhalt einer klaren Lösung weden die weiteren Bestandteile der Phase B zugegeben und dann die Phase B zum Gemisch der Phasen C und D gegeben und homogenisiert. Die Bestandteile der Phase A werden vermengt und erwärmt. Dann wird Phase A unter Homogenisieren zum Gemisch der übrigen Phasen gegeben.

Als Konservierungsmittel werden verwendet:
0,05 % Propyl-4-hydroxybenzoat
0,15 % Methyl-4-hydroxybenzoat

### Beispiel 9

| **Sonnenschutzgel (O/W)** | | **Gew.-%** |
|---|---|---|
| **A** | Eusolex® 6300 (Art.-Nr. 5385) | 0,75 |
| | Luvitol® EHO | 10,00 |
| | Dimeticon | 2,00 |
| | Sheabutter | 5,00 |
| | Antaron® V-220 | 2,00 |
| | Oxynex® K liquid (Art.-Nr. 8324) | 1,00 |
| | | |
| **B** | Verbindung 2 | 1,00 |
| | Eusolex® 232 (Art. Nr. 5372) | 0,75 |
| | Tris(hydroxymethyl)-aminomethan | 0,33 |
| | Konservierungsmittel | q.s. |
| | Wasser, demineralisiert | 20,00 |
| | | |
| **C** | Tris(hydroxymethyl)-aminomethan | 1,20 |
| | Wasser, demineralisiert | 10,00 |
| | | |
| **D** | Pemulen® TR-1 | 0,60 |
| | Wasser, demineralisiert | ad 100,00 |

### Herstellung:

Das Pemulen® TR-1 wird im Wasser der Phase D gelöst. Das Tris(hydroxymethyl)aminomethan wird im Wasser der Phase C gelöst und die Lösung zur Phase D gegeben. Das Tris(hydroxymethyl)aminomethan wird im Wasser der Phase B gelöst und dann unter Rühren das Eusolex® 232 zugegeben. Nach Erhalt einer klaren Lösung werden die weiteren Bestandteile der Phase B zugegeben und dann die Phase B zum Gemisch der Phasen C und D gegeben und homogenisiert. Die Bestandteile der Phase A werden vermengt und erwärmt. Dann wird Phase A unter Homogenisieren zum Gemisch der übrigen Phasen gegeben.

Als Konservierungsmittel wird verwendet:
0,05 % Propyl-4-hydroxybenzoat
0,15 % Methyl-4-hydroxybenzoat

### Beispiel 10

| **Sonnenschutzlotion (W/O) mit UVA/B-Schutz** | | **Gew.-%** |
|---|---|---|
| **A** | Verbindung 7 | 1,00 |
| | Eusolex® 2292 (Art-Nr. 1.05382) | 3,00 |
| | Eusolex® 4360 (Art.-Nr. 1.05376) | 2,00 |
| | Dehymuls® E | 6,00 |
| | gehärtetes Castoröl | 1,00 |
| | Bienenwachs | 2,00 |
| | Oleylerucat | 6,00 |
| | Decylol eat | 6,00 |
| | Dicaprylether | 5,00 |
| | Dimeticon | 1,00 |
| | | |
| **B** | Glycerin (87 %) | 5,00 |
| | Magnesiumsulfat Heptahydrat | 1,00 |
| | Konservierungsmittel | q.s. |
| | Wasser, demineralisiert | ad 100,00 |

Dehymuls® E ist eine Mischung aus Dicodylpentaerythritylcitrat, Sorbitolsesquioleat, Bienenwachs und Aluminiumstearat und wird von der Henkel KGaA, Düsseldorf, DE, vertrieben.

### Herstellung:

Die Bestandteile der Phasen A und B werden jeweils vermengt. Phase A wird auf 75°C und Phase B getrrennt auf 80°C erwärmt. Phase B wird unter Homogenisieren zu Phase A gegeben. Anschließend wird unter Rühren abgekühlt.

Als Konservierungsmittel werden verwendet:
0,05 % Propyl-4-hydroxybenzoat
0,15 % Methyl-4-hydroxybenzoat.

### Beispiel 11

| **Sonnenschutzlotion (W/O) mit UVA/B-Schutz** | | **Gew.-%** |
|---|---|---|
| **A** | Eusolex® 2292 (Art-Nr. 1.05382) | 3,00 |
| | Eusolex® 4360 (Art.-Nr. 1.05376) | 2,00 |
| | Dehymuls® E | 6,00 |
| | gehärtetes Castoröl | 1,00 |
| | Bienenwachs | 2,00 |
| | Oleylerucat | 6,00 |
| | Decyloleat | 6,00 |
| | Dicaprylether | 5,00 |
| | Dimeticon | 1,00 |
| | | |
| **B** | Verbindung 2 | 1,00 |
| | Glycerin (87 %) | 5,00 |
| | Magnesiumsulfat Heptahydrat | 1,00 |
| | Konservierungsmittel | q.s. |
| | Wasser, demineralisiert | ad 100,00 |

### Herstellung:

Die Bestandteile der Phasen A und B werden jeweils vermengt. Phase A wird auf 75°C erwärmt und getrennt davon Phase B auf 80°C. Phase B wird unter Homogenisierung zu Phase A gegeben. Anschließend wird unter Rühren abgekühlt.

Als Konservierungsmittel werden verwendet:
0,05 % Propyl-4-hydroxybenzoat
0,15 % Methyl-4-hydroxybenzoat.

## Patentansprüche

1. Verbindungen der Formel I 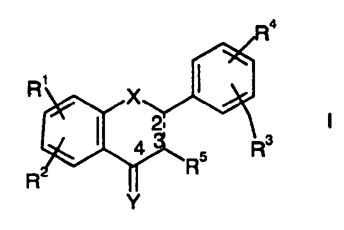 wobei
X steht für O, S oder NH;
Y steht für O, S oder NH;
zwischen den Kohlenstoffen C-2 und C-3 eine Einfach- oder eine Doppelbindung vorgesehen sein kann,
R¹, R², R³, R⁴ und R⁵ gleich oder verschieden sein können und unabhängig voneinander stehen für -H, -OH oder -OA,
und
A steht für eine Gruppe, die im UVA- und/oder UVB-Bereich UV-Strahlung absorbiert, welche ausgewählt ist aus der Gruppe, die gebildet ist aus: 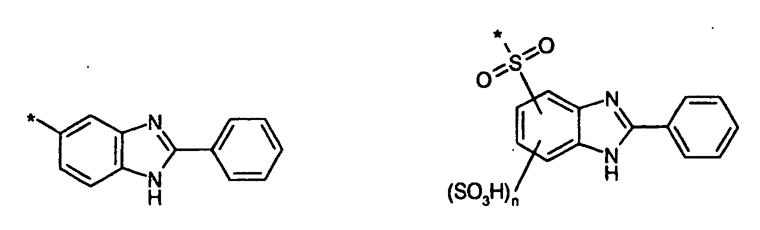 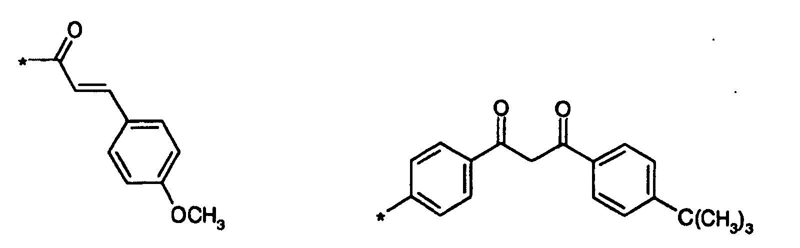 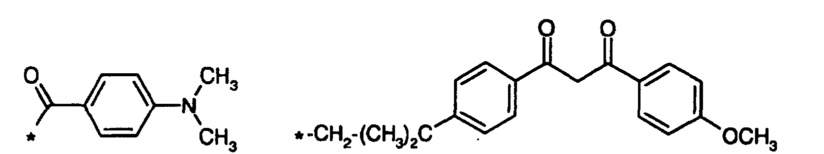 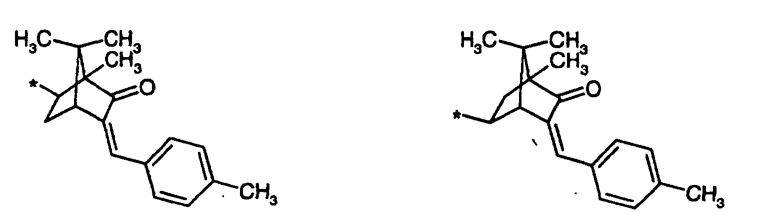 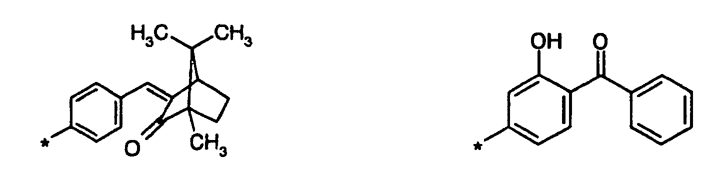 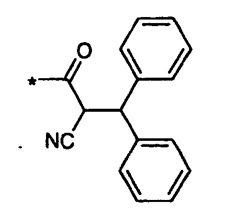 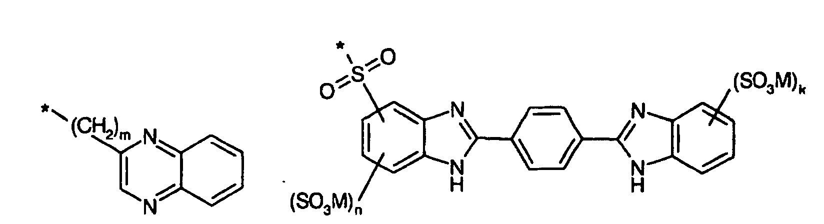 mit n = 0, 1, 2 oder 3
m = 0 oder 1
k = 0, 1, 2, 3 oder 4
M = H, Na oder K
und zumindest eine der Gruppen R¹, R², R³, R⁴ oder R⁵ durch -OA gebildet wird.

2. Verbindung nach Anspruch 1, wobei weiter R¹, R², R³, R⁴ und R⁵ unabhängig voneinander stehen für eine
• geradkettige oder verzweigte Oxyalkyl- oder Carboxyalkylgruppe mit 1 bis 12 Kohlenstoffatomen,
• geradkettige oder verzweigte Oxyalkenyl- oder Carboxyalkenylgruppe mit 2 bis 12 Kohlenstoffatomen,
• geradkettige oder verzweigte Hydroxyoxyalkylgruppe mit 1 bis 12 Kohlenstoffatomen, wobei die Hydroxygruppe an ein primäres oder sekundäres Kohlenstoffatom gebunden sein kann und weiter die Alkylkette auch durch Sauerstoff unterbrochen sein kann,
• Sulfatgruppe,
• Phosphatgruppe
• sowie für einen Mono- oder Oligoglycosylrest.

3. Verbindung nach Anspruch 1 oder 2 der Formel II 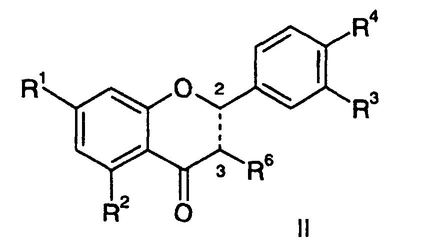 wobei R1, R2, R3, R4 und R5 die in den Ansprüchen 1 und 2 angegebene Bedeutung haben und zwischen den Kohlenstoffen 2 und 3 eine Einfachbindung oder eine Doppelbindung vorgesehen sein kann.

4. Kosmetische oder pharmazeutische Formulierung, enthaltend zumindest eine Verbindung nach Anspruch 1, 2 oder 3.

5. Kosmetische Formulierung nach Anspruch 4, wobei die Formulierung einen oder mehrere zusätzliche UV-Filter und/oder Antioxidanzien enthält.

6. Kosmetische oder pharmazeutische Formulierung nach Anspruch 4 oder 5 zum Schutz von Körperzellen gegen oxidativen Stress, insbesondere zur Verringerung der Hautalterung.

7. Nahrungsmittel, enthaltend zumindest eine Verbindung nach Anspruch 1, 2 oder 3.

8. Verbindungen nach Anspruch 1, 2 oder 3 als Arzneimittel.

9. Verwendung einer Verbindung nach Anspruch 1, 2 oder 3 zur Herstellung eines Arzneimittels gegen oxidativen Stress, insbesondere zur Verminderung der Hautalterung.

10. Verwendung einer Verbindung nach Anspruch 1, 2 oder 3 zur Herstellung eines Arzneimittels zur Behandlung von Entzündungen oder allergischen Reaktionen.

11. Verwendung einer Verbindung nach Anspruch 1, 2 oder 3 als Antioxidationsmittel für kosmetische Formulierungen.

12. Verwendung von Verbindungen nach Anspruch 1, 2 oder 3 zur Stabilisierung von UV-Filtern, insbesondere Dibenzoylmethan und Derivaten des Dibenzoylmethans.

## Claims

1. Compounds of the formula I 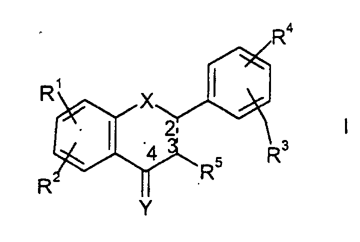 where
X is O, S or NH;
Y is O, S or NH;
a single or double bond may be provided between carbons C-2 and C-3,
R¹, R², R³, R⁴ and R⁵ may be identical or different and independently of one another are -H, -OH or -OA,
and
A is a group which absorbs UV radiation in the UVA and/or UVB region, which is chosen from the group formed from: 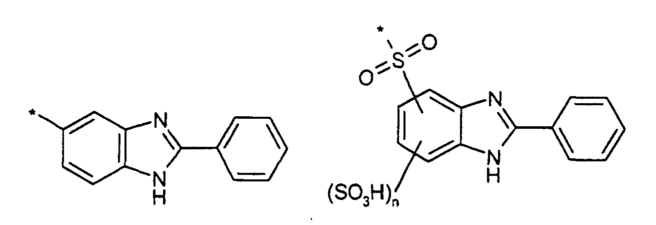 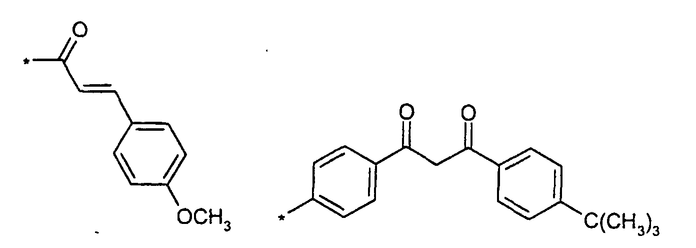 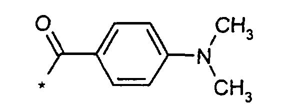 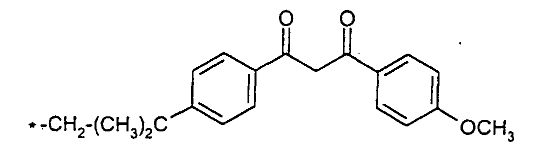 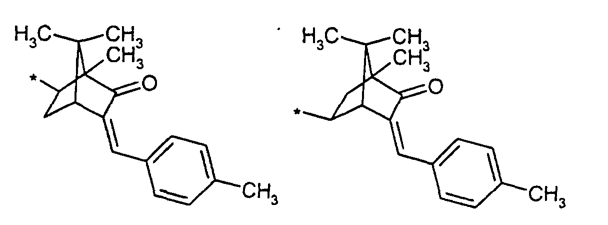 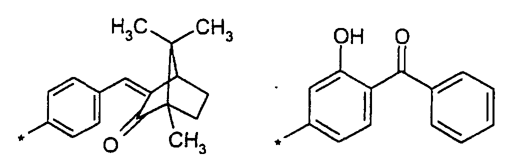 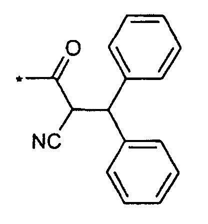 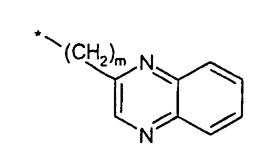 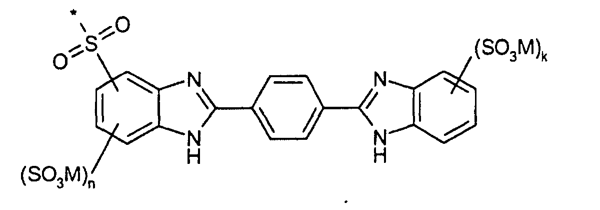 where n = 0, 1, 2 or 3
m = 0 or 1
k = 0, 1, 2, 3 or 4
M = H, Na or K
and at least one of the groups R¹, R², R³, R⁴ or R⁵ is formed by -OA.

2. Compound according to Claim 1, where in addition R¹, R², R³, R⁴ and R⁵, independently of one another, stand for a
• straight-chain or branched oxyalkyl or carboxyalkyl group having 1 to 12 carbon atoms,
• straight-chain or branched oxyalkenyl or carboxyalkenyl group having 2 to 12 carbon atoms,
• straight-chain or branched hydroxyoxyalkyl group having 1 to 12 carbon atoms, where the hydroxyl group may be bonded to a primary or secondary carbon atom and, furthermore, the alkyl chain can also be interrupted by oxygen,
• sulphate group,
• phosphate group
• and a mono- or oligoglycosyl radical.

3. Compound according to Claim 1 or 2 of the formula II 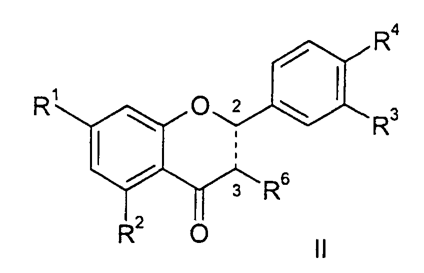 where R¹, R², R³, R⁴ and R⁵ have the meanings given in Claims 1 and 2, and a single bond or a double bond may be provided between carbons 2 and 3.

4. Cosmetic or pharmaceutical formulation comprising at least one compound according to Claim 1, 2 or 3.

5. Cosmetic formulation according to Claim 4, where the formulation comprises one or more additional UV filters and/or antioxidants.

6. Cosmetic or pharmaceutical formulation according to Claim 4 or 5 for protecting the body's cells against oxidative stress, in particular for reducing skin ageing.

7. Foodstuff comprising at least one compound according to Claim 1, 2 or 3.

8. Compounds according to Claim 1, 2 or 3 as medicaments.

9. Use of a compound according to Claim 1, 2 or 3 for the preparation of a medicament against oxidative stress, in particular for reducing skin ageing.

10. Use of a compound according to Claim 1, 2 or 3 for the preparation of a medicament for the treatment of inflammations or allergic reactions.

11. Use of a compound according to Claim 1, 2 or 3 as antioxidant for cosmetic formulations.

12. Use of compounds according to Claim 1, 2 or 3 for the stabilization of UV filters, in particular dibenzoylmethane and derivatives of dibenzoylmethane.

## Revendications

1. Composés de formule I 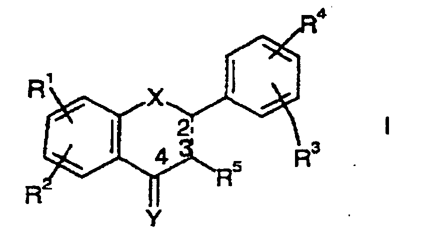 dans lesquels
X est O, S ou NH ;
Y est O, S ou NH ;
une liaison simple ou double pouvant être prévue entre les atomes de carbone en C2 et C3,
R¹, R², R³, R⁴ et R⁵ peuvent être identiques ou différents et sont indépendamment -H, -OH ou -OA,
et
A est un groupe qui absorbe le rayonnement UV dans le domaine UVA et/ou UVB, qui est choisi dans le groupe formé de : 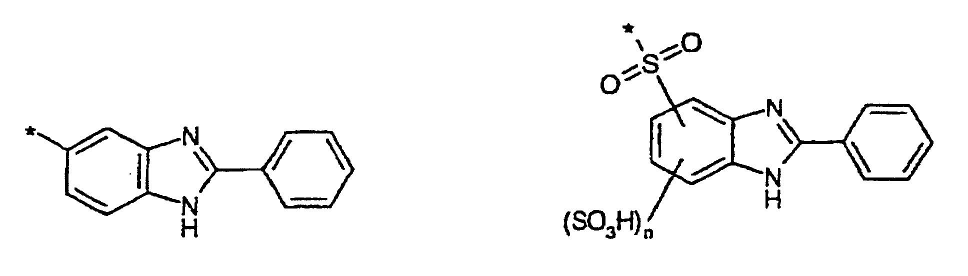 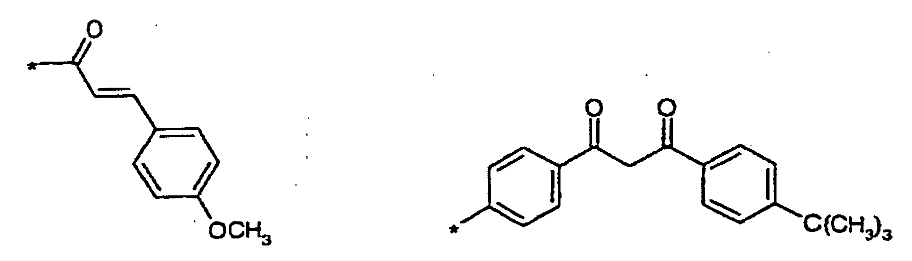 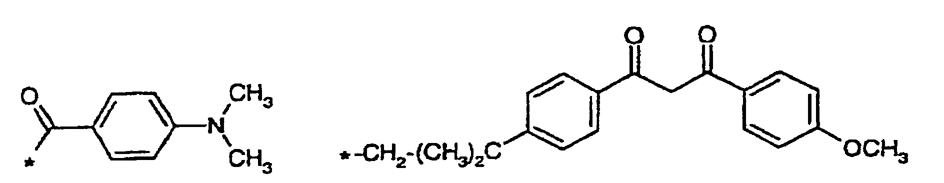 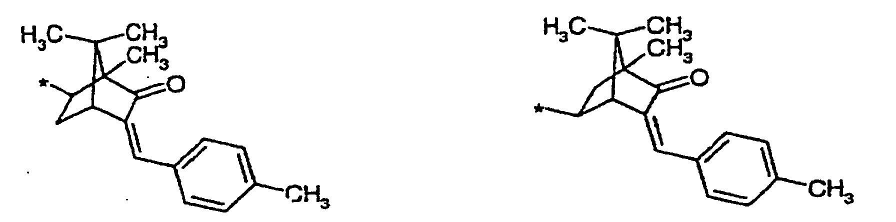 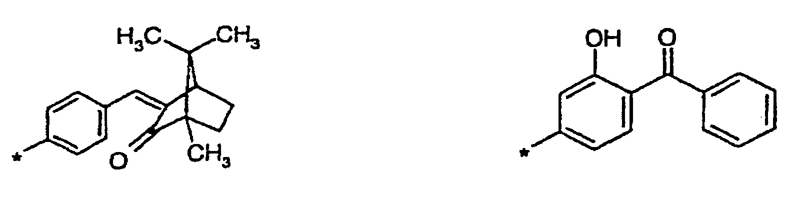 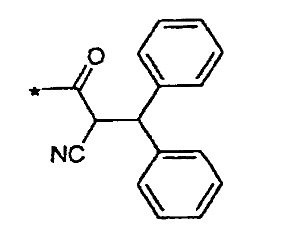 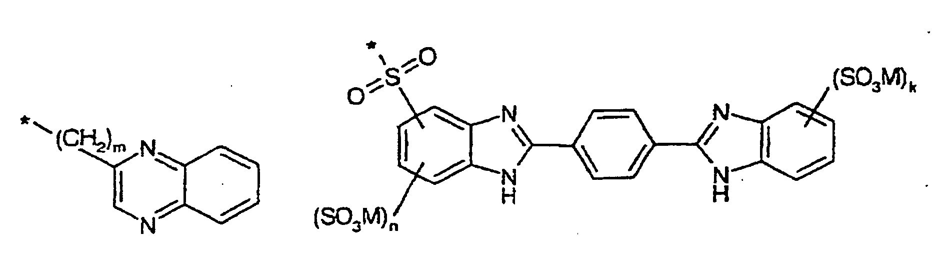 dans lequel n = 0, 1, 2 ou 3
m = 0 ou 1,
k = 0, 1, 2, 3 ou 4,
M = H, Na ou K,
et au moins l'un des groupes R¹, R², R³, R⁴ ou R⁵ est formé par -OA.

2. Composé selon la revendication 1, dans laquelle R¹, R², R³, R⁴ et R⁵ sont indépendamment
* un groupe oxylalkyle ou carboxylalkyle à chaîne linéaire ou ramifiée avec 1 à 12 atomes de carbone,
* un groupe oxylalkényle ou carboxylalkényle à chaîne linéaire
ou ramifiée avec 2 à 12 atomes de carbone,
* un groupe hydroxyoxyalkyle à chaîne linéaire ou ramifiée avec 1 à 12 atomes de carbone, le groupe hydroxy pouvant être lié à un atome de carbone primaire ou secondaire et en outre la chaîne alkyle pouvant être interrompue également par un atome d'oxygène,
* un groupe sulfate,
* un groupe phosphate,
* ainsi qu'un résidu mono- ou oligoglycosyle.

3. Composé selon la revendication 1 ou 2 de formule II 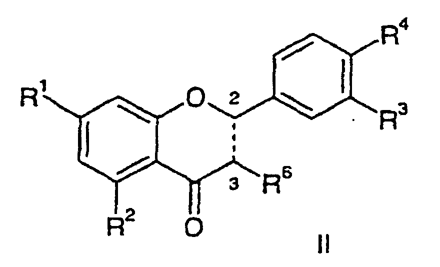 dans lequel R¹, R², R³, R⁴ et R⁵ sont tels que définis dans les revendications 1 et 2 et une liaison simple ou double pouvant être prévue entre les atomes de carbone 2 et 3.

4. Préparation cosmétique ou pharmaceutique, contenant au moins un composé selon la revendication 1, 2 ou 3.

5. Préparation cosmétique selon la revendication 4, la préparation contenant un ou plusieurs filtres UV supplémentaires et/ou antioxydants.

6. Préparation cosmétique ou pharmaceutique selon la revendication 4 ou 5 pour la protection des cellules corporelles contre le stress oxydatif, en particulier pour réduire le vieillissement cutané.

7. Aliment contenant au moins un composé selon la revendication 1, 2 ou 3.

8. Composés selon la revendication 1, 2 ou 3 comme médicaments.

9. Utilisation d'un composé selon la revendication 1, 2 ou 3 pour produire un médicament contre le stress oxydatif, en particulier pour réduire le vieillissement cutané.

10. Utilisation d'un composé selon la revendication 1, 2 ou 3 pour produire un médicament pour le traitement des inflammations ou des réactions allergiques.

11. Utilisation d'un composé selon la revendication 1, 2 ou 3 comme agent anti-oxydant pour des préparations cosmétiques.

12. Utilisation des composés selon la revendication 1, 2 ou 3, pour stabiliser des filtres UV, en particulier le dibenzoylméthane et des dérivés du dibenzoylméthane.
